(19) Europäisches Patentamt
European Patent Office
Office européen des brevets

(11) Publication number: **0 309 028**
**A2**

(12) **EUROPEAN PATENT APPLICATION**

(21) Application number: 88201976.3

(22) Date of filing: 09.09.88

(51) Int. Cl.4: C07D 495/04 , A61K 31/38 , //(C07D495/04,333:00,307:00)

Claims for the following Contracting States: ES + GR.

(30) Priority: 16.09.87 US 97003

(43) Date of publication of application: 29.03.89 Bulletin 89/13

(84) Designated Contracting States: AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: Halczenko, Wasyl
1003 Clymer Road
Hatfield Pennsylvania 19440(US)
Inventor: Hartman, George D.
1529 Tennis Circle
Lansdale Pennsylvania 19446(US)
Inventor: Prugh, John D.
16 Far View Road
Chalfont Pennsylvania 18914(US)

(74) Representative: Hesketh, Alan, Dr.
European Patent Department Merck & Co., Inc. Terlings Park Eastwick Road
Harlow Essex, CM20 2QR(GB)

(54) **Substituted thieno(2,3-B)furan-2-sulfonamides as antiglaucoma agents.**

(57) Thieno[2,3-b]furan-2-sulfonamides are carbonic anhydrase inhibitors useful in the treatment of elevated intraocular pressure and glaucoma.

EP 0 309 028 A2

# SUBSTITUTED THIENO[2,3-B]FURAN-2-SULFONAMIDES AS ANTIGLAUCOMA AGENTS

## SUMMARY OF THE INVENTION

This invention relates to novel aromatic sulfonamides useful in the treatment of elevate intraocular pressure. More particularly this invention relates to compounds having the structural formula:

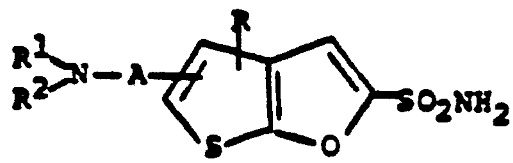

wherein A, R, $R^1$, and $R^2$ are as hereinafter defined, as well as the pharmaceutically and ophthalmologically acceptable salts thereof. This invention also relates to pharmaceutical compositions and the use thereof for systemic and ophtholmic use employing a novel compound of this invention as active ingredient for the treatment of elevated intraocular pressure, especially when accompanied by pathological damage such as in the disease known as glaucoma. The invention also relates to processes for preparation of the novel compounds.

## BACKGROUND OF THE INVENTION

Glaucoma is an ocular disorder associated with elevated intraocular pressures which are too high for normal function and may result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced. Only recently have clinicians noted that many β-adrenergic blocking agents are effective in reducing intraocular pressure. While many of these agents are effective in reducing intraocular pressure, they also have other characteristics, e.g. membrane stabilizing activity, that are not acceptable for chronic ocular use. (S)-1-tert-Butylamino-3-[(4-morpholino-1,2,5-thiadiazol-3-yl)oxy]-2-propanol, a β-adrenergic blocking agent, was found to reduce intraocular pressure and to be devoid of many unwanted side effects associated with pilocarpine and, in addition, to possess advantages over many other β-adrenergic blocking agents, e.g. to be devoid of local anesthetic properties, to have a long duration of activity, and to display minimal tolerance.

Although pilocarpine, physostigmine and the β-blocking agents mentioned above reduce intraocular pressure, none of these drugs manifests its action by inhibiting the enzyme carbonic anhydrase and, thereby, impeding the contribution to aqueous humor formation made by the carbonic anhydrase pathway.

Agents referred to as carbonic anhydrase inhibitors, block or impede this inflow pathway by inhibiting the enzyme, carbonic anhydrase. While such carbonic anhydrase inhibitors are now used to treat intraocular pressure by oral, intravenous or other systemic routes, they thereby have the distinct disadvantage of inhibiting carbonic anhydrase throughout the entire body. Such a gross disruption of a basic enzyme system is justified only during an acute attack of alarmingly elevated intraocular pressure, or when no other agent is effective. Despite the desirability of directing the carbonic anhydrase inhibitor only to the desired ophthalmic target tissue, no topically effective carbonic anhydrase inhibitors are available for clinical use.

However, topically effective carbonic anhydrase inhibitors are reported in U.S. Patents 4,386,098; 4,416,890; and 4,426,388. The compounds reported therein are 5 (and 6)-hydroxy-2-benzothiazolesulfonamides and acyl esters thereof.

## DETAILED DESCRIPTION OF THE INVENTION

## DETAILED DESCRIPTION OF THE INVENTION

The novel compounds of this invention have structural formula:

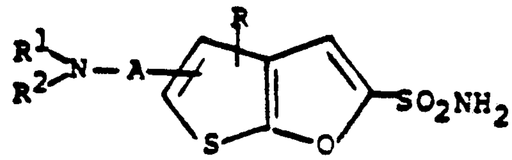

or a pharmaceutically acceptable salt thereof, wherein
A is $C_{1-8}$ alkylene, either straight or branched chain and either unsubstituted or substituted with $C_{1-3}$ alkoxy or hydroxy; and
R is hydrogen or $C_{1-6}$alkyl, either straight or branched chain; and
$R^1$ and $R^2$ are independently:

1) hydrogen,
2) $C_{1-6}$ alkyl, either unsubstituted or substituted with one or more of
   a) $C_{1-3}$ alkoxy,
   b) $C_{1-3}$alkoxy-$C_{2-4}$alkoxy,
   c) hydroxy,
   d) phenyl,
   e) halo such as chloro, bromo or fluoro,
   f) heteroaromatic such as pyridyl, pyrimidinyl, pyrazinyl or imidazolyl, or
   g) $-NR^3R^4$ wherein $R^3$ and $R^4$ are independently selected from

i) hydrogen and
ii) $C_{1-6}$ alkyl, either unsubstituted or substituted with one or more of $C_{1-3}$ alkoxy, hydroxy or phenyl, or
   h) $-SR^3$,

$-\overset{O}{S}-R^3$, $-SO_2R^3$; or

3) taken together with the nitrogen atom to which they are attached form a 5 to 7-membered heterocycle such as piperidine, morpholine, thiomorpholine, thiomorpholine -S-oxide, thiomorpholine-S,S-dioxide, piperazine, or N-$C_{1-3}$ alkylpiperazine.

A preferred embodiment of the novel compounds is that wherein A is joined to the 5-position of the thieno[2,3-b]furan ring system.

It is still more preferred that A is $-(CH_2)_{1-3}$, especially $-CH_2$.

The novel processes for preparing the novel compounds of this invention are illustrated as follows, wherein Ar represents

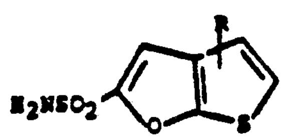

and A′ represents $C_{0-7}$alkylene either unsubstituted or substituted with $C_{1-3}$alkoxy or hydroxy:

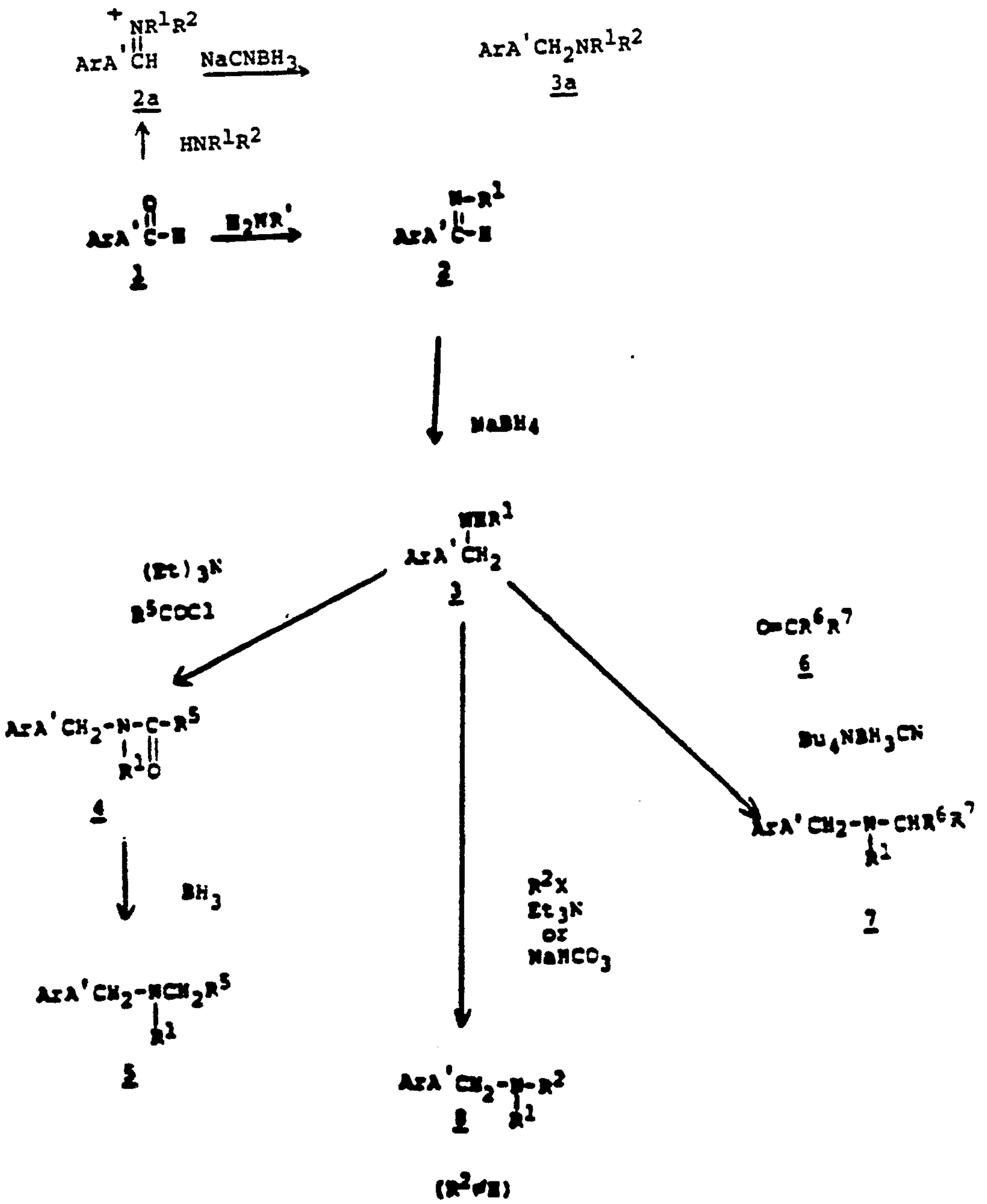


The process comprises treating the aldehyde, 1, in the presence of a slight excess of HCl gas with the amine $H_2NR^1$ or $HNR^1R^2$ at about 15 to 60° C in a $C_{1-3}$ lower alkanol solvent such as methanol or ethanol for about 0.5 to 4 hours. The crystalline precipitate of 2 or 2a is redissolved by addition of THF or isolated and dissolved in methanol and THF and treated wtih a complex metal hydride such as sodium borohydride in the case of 2 or sodium cyanoborohydride in the case of 2a at about 0 to 20° C and continuing stirring for about 0.5 to 6 hours.

Compound 3 is converted into a tertiary amine either by acylation to 4 and reduction to 5, or by treatment with ketone 6, followed by reduction to give 7. Conversion of 3 to 4 would be carried out in an aprotic solvent such as ether, THF, or the like in the presence of a base such as triethylamine at about room temperature and the subsequent reduction to 5 by $BH_3 \bullet S(CH_3)_2$ would be carried out at about room temperature in a solvent such as toluene, $CH_2Cl_2$ or THF.

Conversion of 3 to 7 would be carried out by treatment of 3 with an equivalent amount of ketone 6 at about room temperature in a solvent such as a halocarbon (methylene chloride) or an alcohol, such as methanol.

Alternatively, secondary amine 3 can be converted into tertiary amine 8 simply by alkylation. Thus, treatment of 3 in ether, THF, DMF or halocarbon solution with an alkyl halide, $R^2X$, (iodide bromide or

chloride) in the presence of an organic ($(C_2H_5)_3N$) or inorganic ($NaHCO_3$) base would give 8.

Those novel compounds wherein A is branched chain alkylene can be prepared either from the aldehyde as shown in the above reaction schemes wherein A′ is a branched chain alkylene, or from the aromatic aldehyde as shown below:

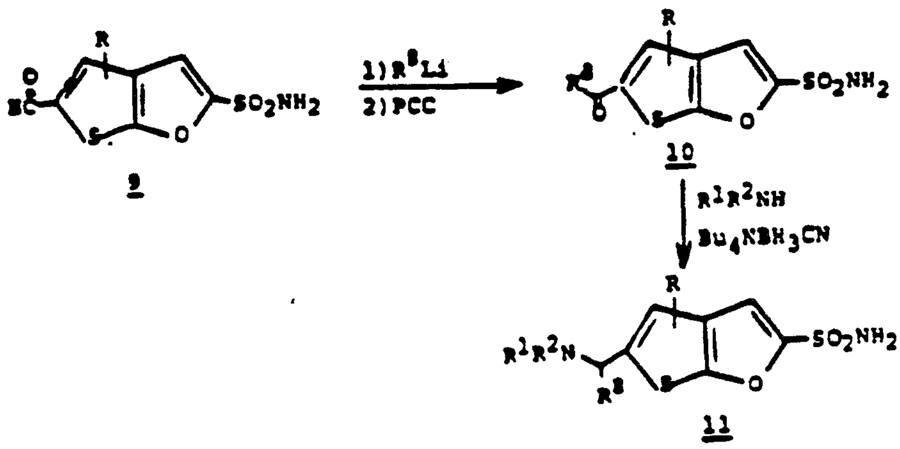


9 10 11

In this process 9 is treated with organolithium reagent $R^8Li$ in ether or THF at -78° to -30° C, this is quenched with $H_2O$ and the product extracted. This alcohol is oxidized to ketone 10 with pyridinium chlorochromate (PCC) in $CH_2Cl_2$ at room temperature (2-3 hours). The ketone 10 is reductively aminated with amine $R^1R^2NH/Bu_4NBH_3CN$ in an alcohol ($CH_3OH$) at room temperature for 1-5 hours to provide 11. In this manner the following compounds are made:

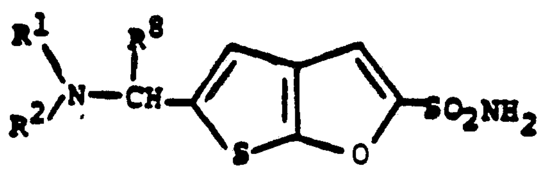

| $R^1$ | $R^2$ | $R^8$ |
|---|---|---|
| $CH_3-$ | $C_2H_5-$ | $CH_3-$ |
| $CH_3OCH_2CH_2-$ | H | $C_2H_5-$ |
| $C_6H_5CH_2-$ | $HOCH_2CH_2-$ | $(CH_3)_2CH-$ |
| $CH_3OCH_2CH_2-$ | $CH_3OCH_2CH_2-$ | $CH_3-$ |
| $CH_3OCH_2CH_2-$ | $HOCH_2CH_2$ | $CH_3OCH_2CH_2-$ |
| $FCH_2CH_2-$ | $CH_3OCH_2CH_2$ | $CH_3CH_2-$ |
| $---(CH_2)_2-O---(CH_2)_2---$ | | $CH_3OCH_2CH_2-$ |
| $---(CH_2)_2-S(O)---(CH_2)_2---$ | | $CH_3$ |

The novel pharmaceutical formulations of this invention are adapted for topical ocular administration in the form of solutions, ointments, solid water soluble polymeric inserts, or solutions which gel at body temperature or in the presence of lachrymal fluids for the treatment of glaucoma and other stages of elevated intraocular pressure and contain about 0.1% to 15% by weight of medicament, especially about 0.5 to 2% by weight of medicament, the remainder being comprised of carriers and other excipients well known in the art.

The medicament in the novel topical ocular formulations comprises one of the novel compounds of this invention either alone or in combination with a β-adrenergic blocking agent such as timolol maleate or a parasympathomimetic agent such as pilocarpine. In such combinations the two active agents are present in approximately equal amounts.

The novel method of treatment of this invention comprises the treatment of elevated intraocular pressure by the administration of an effective amount of a novel compound of this invention or a pharmaceutical formulation thereof. Of primary concern is the treatment by topical ocular administration of about 0.1 to 25 mg and especially 0.2 to 10 mg of such compound per day, either by single dose or on a 2 to 4 dose per day regimen.

## EXAMPLE 1

### 5-(Isobutylaminomethyl)thieno[2,3-b]furan-2-sulfonamide hydrochloride

#### Step A: Preparation of 2-(Carbomethoxymethylthio)-3-[2-(1,3-dioxolanyl)]furan

To a mechanically stirred solution of 69.0 g (0.49 mol) of 3-[2-(1,3-dioxolanyl)furan in 250 ml of THF under $N_2$ and cooled to -78°C was added 0.49 mol of n-butylithium (in hexane) dropwise at <-70°C. This was stirred at -78°C for 40 minutes to provide a white suspension. Sulfur (16.64 g, 0.52 mol) was added via Gooch tubing portionwise as the temperature rose to -65°C to provide an orange suspension which was stirred at -78°C for 0.5 hours and then at -50°C for 0.5 hour to afford a deep purple reaction mixture. Methyl bromoacetate 91.8 g (0.60 mol) in 50 ml THF was added dropwise at -78°C to give a brownish suspension that was stirred at -70°C for 0.5 hour and then was allowed to gradually warm to 0°C over 1.5 hours.

The reaction mixture was quenched with a mixture of 250 ml brine/250 ml ether. The aqueous phase was separated, re-extracted with 200 ml of ether and the organic phases were combined, washed with brine, dried and evaporated to dryness in vacuo. The resulting oil was taken up in ether and passed through a silica gel pad to provide a clear filtrate. The solvent was removed in vacuo to give the title compound as an oil: $R_f$ 0.4 on silica gel eluting with 20% ethyl acetate/hexanes.

#### Step B: Preparation of 2-(Carbomethoxymethylthio)-furan-3-carboxaldehyde

To a solution of 6.8 g (0.028 mol) of product from Step A dissolved in 50 ml acetone was added 100 mg p-toluenesulfonic acid monohydrate and the resulting solution was kept at room temperature for 3.0 hours. Saturated $NaHCO_3$ solution (15 ml) was added and then resulting suspension was stripped in vacuo. The residue was extracted with 2 x 40 ml portions of ether and the combined organic extracts were washed with brine and dried. The solvent was removed in vacuo to give the title compound as a clear oil: $R_f$ 0.5 on silica gel eluting with 20% ethyl acetate/hexanes.

#### Step C: Preparation of Methyl Thieno[2,3-b]furan-5-carboxylate

To a solution of 89.0 g (0.58 mol) 1,8-diazabicyclo[5.4.0]undec-7-ene in 1.5 l of THF at room temperature was added 78.5 g (0.39 mol) of product from Step B in 500 ml THF dropwise and the resulting solution was stirred for 24 hours. The THF solution was decanted from the black, tarry residue and the solvent was evaporated in vacuo. The residue was taken up in ether, washed with water, brine and dried. The solvent was removed in vacuo to give a residue that was triturated with 50 ml of 50% 2-propanol/hexane to give a pale yellowish solid. The filtrate was evaporated and the residue was purified by flash chromatography on silica gel eluting with 8% ethyl acetate/hexane to afford pure title compound, $R_f$ 0.4. This was combined with the solid isolated initially after trituration with hexane, to give title compound as a white solid, m.p. 86-88°C.

Step D: Preparation of Thieno[2,3-b]furan-5-carbinol

To a suspension of 3.80 g (0.1 mol) of lithium aluminum hydride in 500 ml ether cooled to 0-10 °C was added a solution of 9.1 g (0.05 mol) of product from Step C in 150 ml ether dropwise over 20 minutes. This suspension was stirred at room temperature for 4 hours and was then cooled and quenched by dropwise addition of 30 ml of saturated Na+/K+ tartrate solution. This suspension was stirred at room temperature for 0.5 hour and the ether phase was decanted from the gummy solid. This solid was triturated with ether and the combined organic phases were washed with brine and dried. The solvent was removed in vacuo to provide the title compound as an oil: $R_f$ 0.3 on silica gel eluting with 20% ethyl acetate/hexane.

Step E: Preparation of Thieno[2,3-b]furan-5-carboxaldehyde

To a stirred suspension of 1.43 g (6.6 mmol) of pyridinium chlorochromate in 15 ml methylene chloride at room temperature was added 0.68 g (4.4 mmol) of product from Step D in 15 ml methylene chloride in one portion and the resulting dark suspension was stirred at room temperature for 2 hours. The reaction mixture was diluted with 20 ml $CH_2Cl_2$ and was filtered through a silica gel pad. The solvent was removed in vacuo to give the title compound as a viscous yellow oil: $R_f$ 0.4 on silica gel eluting with 20% ethyl acetate/hexane.

Step F: Preparation of 5-[2-(1,3-Dioxolanyl)]thieno[2,3-b]furan

To a solution of 5.91 g (0.029 mol) of product from Step E and 4.96 g (0.08 mol) ethylene glycol in 75 ml benzene was added 100 mg pyridinium p-toluenesulfonate and the resulting solution was heated at reflux utilizing a Dean-Stark trap. After 5 hours all starting material was consumed. The cooled reaction mixture was diluted with 75 ml water and the organic phase was separated and dried. This was filtered through a pad of silica gel and the solvent was removed in vacuo to give the title compound as an oil: $R_f$ 0.6 on silica gel eluting with 20% ethyl acetate/hexane.

Step G: Preparation of 5-[2-(1,3-Dioxolanyl)]thieno[2,3-b]furan-2-sulfonamide

To a solution of 1.96 g (0.01 mol) of product from step F in 20 ml THF cooled to -78 °C under $N_2$ was added 0.01 mol n-butyllithium dropwise at <-70 °C and the resulting solution was stirred for 45 minutes at -78 °C. Sulfur dioxide gas was introduced close to the surface of the reaction mixture as the temperature rose to about -60 °C. The suspension was stirred at -65 °C for 0.5 hours while $SO_2$ gas was continuously admitted and then allowed to gradually warm to 0 °C over 1.0 hour. The solvent was removed at <35 °C at water aspiration pressure to afford a tan solid. This solid was taken up in 20 ml of saturated $NaHCO_3$ solution and with cooling to 0-10 °C, 2.0 g (0.015 mol) of N-chlorosuccinimide was added portionwise over 5 minutes. The resulting suspension was stirred vigorously at 0-10 °C for 1.5 hours. This was extracted wtih 3 x 50 ml portions of $CHCl_3$ and the combined organic extracts were washed with brine and dried. The solvent was removed in vacuo to give the intermediate sulfonyl chloride as a tan solid. This was dissolved in 15 ml acetone and with cooling to 0-10 °C, 15 ml $NH_4OH$ solution was added in one portion. This was stirred at 0-10 °C for 1.5 hours and then extracted with 5 x 50 ml portions of ethyl acetate. The combined organic extracts were washed with brine, dried and the solvent removed in vacuo to give the title compound as a tan solid.

Step H: Preparation of 5-Formylthieno[2,3-b]furan-2-sulfonamide

To a solution of 1.66 g (0.006 mol) of product from Step G in 35 ml acetone was added about 100 mg p-toluenesulfonic acid monohydrate and the resulting solution was kept at room temperature for 2 hours. This was treated with 25 ml of a saturated $NaHCO_3$ solution and the organic solvent was removed in vacuo. The residue was extracted with 5 x 50 ml portions of ethyl acetate and the combined organic layers were washed with brine and dried. The solvent was removed in vacuo to give the title compound as a tan solid: $R_f$ 0.5 on silica gel eluting with 50% ethyl acetate/hexane, m.p. 165-167 °C.

Step I: Preparation of 5-(Isobutylaminomethyl)thieno[2,3-b]furan-2-sulfonamide hydrochloride

To a suspension of 0.465 g (2.0 mmols) of product from Step H in 5 ml ethanol at room temperature was added 1.02 g (14.0 mmols) of isobutylamine and the resulting solution was stirred at room temperature for 1.0 hour. Then, 0.30 g (8.0 mmols) of sodium borohydride was added at room temperature and this suspension was stirred for 2 hours. The reaction mixture was then poured into 75 ml water and was acidified with 6 N HCl. This acidic mixture was neutralized with aqueous ammonium hydroxide to pH 8-9 and extracted with 4 x 50 ml portions of ethyl acetate. The organic extracts were combined, washed with brine and dried. The solvent was removed in vacuo to give the free base of the title compound as a pale yellow residue. This was purified by flash chromatography on silica gel eluting with 5% methanol/chloroform to provide pure free base as a white solid: $R_f$ 0.3 on silica gel eluting with 5% methanol/chloroform. This was dissolved in 10 ml ethanol, treated with ethanolic HCl and the resulting solution was gradually diluted with 30 ml ether while being cooled in an ice bath to provide the title compound as a white solid, m.p. 237-242 °C.

Employing the procedures substantially as described in Example 1, Step I but substituting for the isobutylamine used therein approximately equimolar portions of the amines of structure $R^1R^2NH$ depicted in Table I there are produced the $R^1R^2$-aminomethylthieno[2,3-b]furan-2-sulfonamides, also depicted in Table I:

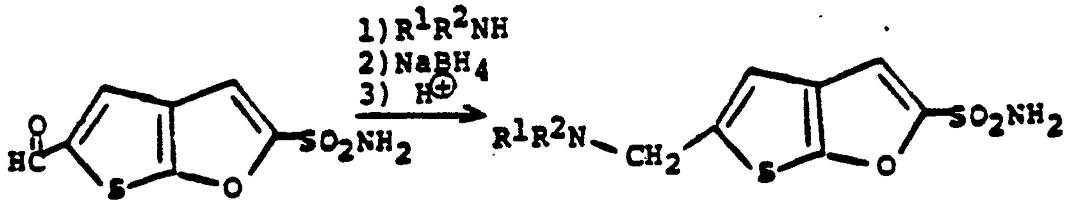


## TABLE I

| $R^2$ | $R^1$ | melting or decomp point ( °C) |
|---|---|---|
| H | $CH_3-$ | 220-223 (HCl) |
| H | $C_2H_5-$ | 225-229 (dec) (HCl) |
| H | 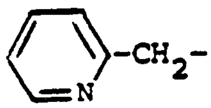 | 149-151 (maleate) |
| H | $HO(CH_2)_4-$ | 218-220 (HCl) |
| H | $CH_3OCH_2CH_2-$ | 190-192° (HCl) |
| H | $CH_3OCH_2CH_2CH_2-$ | 203-205° (HCl) |
| H | $C_2H_5O(CH_2)_2-$ | 200.5-202.5 HCl) |
| H | $CH_3O(CH_2)_2O(CH_2)_3-$ | 182-184 (HCl) |
| H | $n-C_3H_7$ | 251-254 (HCl) |
| $-CH_2CH-_2O-CH_2CH_2-$ | * | 243-246 (HCl) |

| $R^2$ | $R^1$ | | melting or decomp point ( °C) |
|---|---|---|---|
| $CH_3OCH_2CH_2-$ | $CH_3OCH_2CH_2-$ | * | 95-97 (maleate) |
| H | $FCH_2CH_2-$ | | 202-204 (HCl) |

* Intermediate iminium compound was reduced with sodium cyanoborohydride rather than sodium borohydride.

Employing the procedures described in the foregoing the compounds depicted in the following Table also are prepared from the appropriate starting materials.

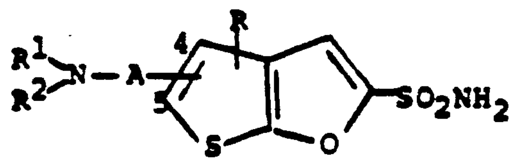

| A | $R^1$ | $R^2$ | R |
|---|---|---|---|
| $5-CH_2-$ | $CH_3-$ | $CH_3-$ | H- |
| $4-CH_2-$ | $CH_3-$ | $CH_3-$ | H- |
| $5-CH_2-$ | $C_2H_5-$ | $C_6H_5CH_2-$ | H- |
| $5-CH_2-$ | H- | $C_2H_5-$ | $4-CH_3-$ |
| $5-CH_2CH_2-$ | $CH_3-$ | $CH_3-$ | H- |
| $5-CH_2CH_2-$ | $i-C_4H_9-$ | H- | $4-CH_3-$ |
| $4-CH_2-$ | $CH_3-$ | $CH_3OCH_2CH_2-$ | H- |
| $4-CH_2-$ | $i-C_4H_9$ | H | H |
| $4-CH_2-$ | $-CH_2CH_2-CH_2-CH_2CH_2-$ | | H |
| $4-CH_2-$ | $-CH_2CH_2-N-CH_2CH_2-$<br>$CH_3$ | | H |
| $4-CH_2-$ | $(CH_3)_2NCH_2CH_2-$ | H | H |

EXAMPLE 2

| | | |
|---|---|---|
| 5-(Isobutylaminomethyl)thieno-[2,3-b]furan-2-sulfonamide hydrochloride | 1 mg | 15 mg |
| Monobasic sodium phosphate•$2H_2O$ | 9.38 mg | 6.10 mg |
| Dibasic sodium phosphate•$12H_2O$ | 28.48 mg | 16.80 mg |
| Benzalkonium chloride | 0.10 mg | 0.10 mg |
| Water for injection q.s. ad. | 1.0 ml | 1.0 ml |

The novel compound, phosphate buffer salts, and benzalkonium chloride are added to and dissolved in water. The pH of the composition is adjusted to 5.4-7.4 and diluted to volume. The composition is rendered sterile by ionizing radiation.

EXAMPLE 3

5-(Isobutylaminomethyl)thieno-[2,3-b]furan-2-sulfonamide hydrochloride 5 mg
petrolatum q.s. ad. 1 gram

The compound and the petrolatum are aseptically combined.

EXAMPLE 4

5-(Isobutylaminomethyl)thieno-[2,3-b]furan-2-sulfonamide hydrochloride 1 mg
Hydroxypropylcellulose q.s. 12 mg

Ophthalmic inserts are manufactured from compression molded films which are prepared on a Carver Press by subjecting the powdered mixture of the above ingredients to a compressional force of 12,000 lbs. (gauge) at 300° F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30° C) for two to four days. After removal from the humidity cabinet, the vials are stoppered and then capped. The vials containing the hydrate insert are then autoclaved at 250° F for 1/2 hour.

## Claims

1. A compound of structural formula:

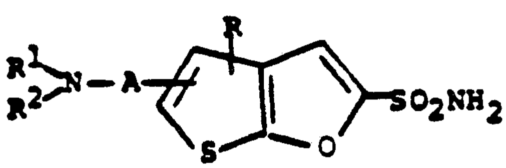

or pharmaceutically acceptable salt thereof wherein
A is $C_{1-8}$ alkylene, either straight or branched chain and either unsubstituted or substituted with $C_{1-3}$ alkoxy or hydroxy; and
R is hydrogen or
$C_{1-6}$ alkyl either straight or branched chain;
$R^1$ and $R^2$ are independently:
1) hydrogen,
2) $C_{1-6}$ alkyl, either unsubstituted or substituted with one or more of
a) $C_{1-3}$ alkoxy,
b) $C_{1-3}$alkoxy-$C_{2-4}$alkoxy,
c) hydroxy,
d) phenyl, or
e) halo,

f) pyridyl, pyrimidinyl, pyazinyl or imidazolyl, or
g) $-NR^3R^4$ wherein $R^3$ and $R^4$ are independently selected from
i) hydrogen and
ii) $C_{1-5}$ alkyl, either unsubstituted or substituted with one or more of $C_{1-3}$ alkoxy, hydroxy or phenyl, or
h) $-SR^3$,
$-S-\overset{O}{\overset{|}{S}}-R^3$ or $-SO_2R^3$; or

3) taken together with the nitrogen atom to which they are attached form a 5 to 7-membered heterocycle.

2. The compound of Claim 1 or a pharmaceutically acceptable salt thereof wherein $R^1R^2N-A-$ is joined to the 5-position of the thieno[2,3-b] furan ring system.

3. The compound of Claim 2, wherein A is $-CH_2-$.

4. The compound of Claim 3, wherein $R^2$ is hydrogen and $R^1$ is $C_{1-6}$ alkyl.

5. The compound of Claim 3 which is 5-Isobutylaminomethylthieno[2,3-b]furan-2-sulfonamide or ophthalmologically acceptable salt thereof.

6. The compound of Claim 3 which is 5-Methylaminomethylthieno[2,3-b]furan-2-sulfonamide or ophthalmoligocally acceptable salt thereof.

7. The compound of Claim 3 which is 5-(2-Methoxyethylaminomethyl)thieno[2,3-b]furan-2-sulfonamide or ophthalmologically acceptable salt thereof.

8. The compound of Claim 3 which is 5-Ethylaminomethylthieno[2,3-b]furan-2-sulfonamide or ophthalmologically acceptable salt thereof.

9. The compound of Claim 3 which is 5-(4-Hydroxybutylaminomethyl)thieno[2,3-b]furan-2-sulfonamide or ophthalmologically acceptable salt thereof.

10. The compound of Claim 3 which is 5-(2-Pyridylaminomethyl)thieno[2,3-b]furan-2-sulfonamide or ophthalmologically acceptable salt thereof.

11. The compound of Claim 3 which is 5-(3-Methoxypropylaminomethyl)thieno[2,3-b]furan-2-sulfonamide or ophthalmologically acceptable salt thereof.

12. The compound of Claim 3 which is
5-(2-Ethoxyethylaminomethyl)thieno[2,3-b]furan-2-sulfonamide
5-(Methoxyethoxyethylaminomethyl)thieno[2,3-b]-furan-2-sulfonamide
5-(Propylaminomethyl)thieno[2,3-b]furan-2-sulfonamide
5-(Morpholinomethyl)thieno[2,3-b]furan-2-sulfonamide
5-[(Bis-2-Methoxyethyl)aminomethyl]thieno[2,3-b]furan-2-sulfonamide or
5-(2-Fluoroethylaminomethyl)thieno[2,3-b]furan-2-sulfonamide or an ophthalmologically acceptable salt thereof.

13. An ophthalmological formulation for the treatment of ocular hypertension and glaucoma comprising an ophthalmologically acceptable carrier and an effective ocular antihypertensive amount of the compound of Claim 1.

14. The use of a compound of Claim 1 for the preparation of a medicament useful for treating ocular hypertension and glaucoma.

Claims for the following Contracting States: ES, GR.

1. A process for preparing substituted thieno [2,3-b]-furan-2-sulfonamides of formula I

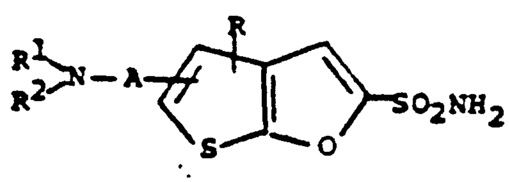

(I)

or pharmaceutically acceptable salt thereof wherein
A is $C_{1-8}$ alkylene, either straight or branched chain and either unsubstituted or substituted with $C_{1-3}$ alkoxy or hydroxy; and
R is hydrogen or

$C_{1-6}$ alkyl either straight or branched chain;

$R^1$ and $R^2$ are independently:

1) hydrogen,

2) $C_{1-6}$ alkyl, either unsubstituted or substituted with one or more of

a) $C_{1-3}$ alkoxy,

b) $C_{1-3}$alkoxy-$C_{2-4}$alkoxy,

c) hydroxy,

d) phenyl, or

e) halo,

f) pyridyl, pyrimidinyl, pyazinyl or imidazolyl, or

g) $-NR^3R^4$ wherein $R^3$ and $R^4$ are independently selected from

i) hydrogen and

ii) $C_{1-6}$ alkyl, either unsubstituted or substituted with one or more of $C_{1-3}$ alkoxy, hydroxy or phenyl, or

h) $-SR^3$,

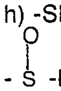 $-R^3$ or $-SO_2R^3$; or

3) taken together with the nitrogen atom to which they are attached form a 5 to 7-membered heterocycle, which comprises:

A) reacting an aldehyde of formula (1)

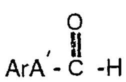 (1)

wherein Ar represents

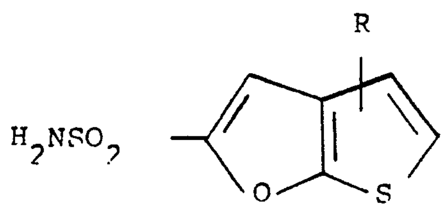

wherein R is that previously defined;

and

A′ represents $C_{0-7}$ alkylene either unsubstituted or substituted with $C_{1-3}$ alkoxy or hyhdroxy;

in the presence of an acid and a solvent with an amine selected between:

A.1) $HNR^1R^2$, wherein $R^1$ and $R^2$ are those previously defined, to render a compound of formula J, after reducing the intermediate iminium compound with a suitable reducing agent such as a complex metal hydride, preferably sodium cyanoborohydride; or

A.2) $H_2NR^1$, wherein $R^1$ is that previously defined to render the corresponding secondary amine of formula (3)

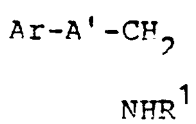

(3)

wherein Ar, A′ and $R^1$ are those previously defined, after reducing the intermediate iminium compound with a suitable reducing agent such as a complex metal hydride, preferably sodium borohydride; said secondary amine of formula 3 may be converted into a tertiary amine by

i) acylation with an acylating agent in the presence of an aprotic solvent, followed by reduction of the N-acyl derivative obtained; or

ii) treatment with a ketone followed by reduction; or

iii) alkylation with an alkyl halide in the presence of a base; or

alternatively,

B) For the preparation of compounds I in which A is branched chain alkylene,

1) reacting an aldehyde of formula (9)

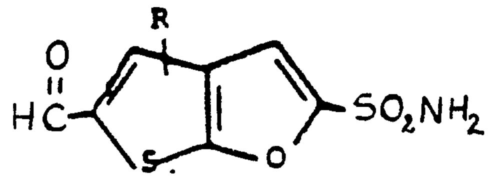

9

wherein R is that previously defined, with an organolithium agent $R^8Li$ (wherein $R^8$ is $C_{1-3}$ alkyl, either straight or branched chain and either substituted with alkoxy or unsubstituted) in the presence of an inert organic solvent to render the corresponding alcohol which is further oxidized with a suitable oxidizing agent, such as PCC in an inert organic solvent, to render the corresponding ketone of formula (10)

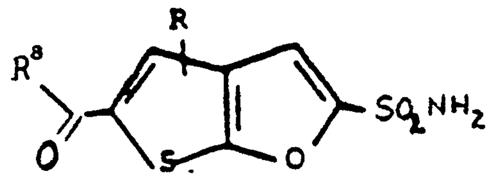

(10)

wherein R and $R^8$ are those previously defined, which is further,

2) reductively aminated with an amine of formula

$R^1R^2NH$

wherein $R^1$ and $R^2$ are those previously defined, in the presence of $Bn_4NBH_3CN$ in an organic solvent to render a compound I in which A is branched chain alkylene of formula

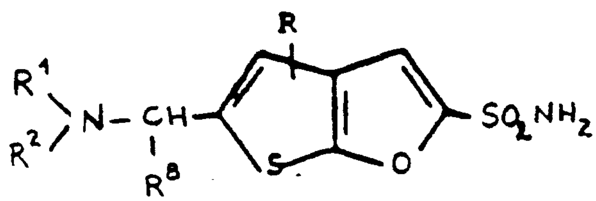

wherein R, $R^1$, $R^2$ and $R^8$ are those previously defined.

2.- The process of claim 1 wherein $R^1R^2N$-A- is joined to the 5-position of the thieno[2,3-b]furan ring system.

3.- The process of claim 2, wherein A is $-CH_2-$.

4.- The process of claim 3, wherein $R^2$ is hydrogen and $R^1$ is $C_{1-6}$alkyl.

5.- The process of claim 3 wherein the compound obtained is 5-Isobutylaminomethylthieno[2,3-b]furan-2-sulfonamide or ophthalmologically acceptable salt thereof.

6.- The process of claim 3 wherein the compound obtained is 5-Methylaminoethylthieno[2,3-b]furan-2-sulfonamide or ophthalmologically acceptable salt thereof.

7.- The process of claim 3 wherein the compound obtained is 5-(2-Methoxyethylaminoethyl)thieno[2,3-b]furan-2-sulfonamide or ophthalmologically acceptable salt thereof.

8.- The process of claim 3 wherein the compound obtained is 5-Ethylaminomethylthieno[2,3-b]furan-2-sulfonamide or ophthalmologically acceptable salt thereof.

9.- The process of claim 3 wherein the compound obtained is 5-(4-Hydroxybutylaminoethyl)thieno[2,3-b]furan-2-sulfonamide or ophthalmologically acceptable salt thereof.

10.- The process of claim 3 wherein the compound obtained is 5-(2-Pyridylaminomethyl)thieno[2,3-b]-furan-2-sulfonamide or ophthalmologically acceptable salt thereof.

11.- The process of claim 3 wherein the compound obtained is 5-(3-Methoxypropylaminomethyl)thieno-[2,3-b]furan-2-sulfonamide or ophthalmologically acceptable salt thereof.

12.- The process of claim 3 wherein the compound obtained is selected from:
5-(2-Ethoxyethylaminomethyl)thieno[2,3-b]furan-2-sulfonamide
5-(Methoxyethoxyethylaminomethyl)thieno[2,3-b]furan-2-sulfonamide
5-(Propylaminomethyl)thieno[2,3-b]furan-2-sulfonamide
5-(Morpholinomethyl)thieno[2,3-b]furan-2-sulfonamide
5-[(Bis-2-Methoxyethyl)aminomethyl]thieno[2,3-b]furan-2-sulfonamide or
5-(2-Fluoroethylaminomethyl)thieno[2,3-b]furan-2-sulfonamide or an ophthalmologically acceptable salt thereof.